# EUROPEAN PATENT SPECIFICATION

(11) **EP 2 862 624 B1**
(45) Date of publication and mention of the grant of the patent: **14.02.2018**
(21) Application number: 13838764.2
(22) Date of filing: 17.06.2013
(51) Int. Cl.: B09B 3/00, B09C 1/00, B01F 17/00, C02F 1/26, C09K 3/32, A61K 8/97, A61K 36/899, C02F 103/26, C02F 103/32, A61Q 19/00, B09C 1/02, C02F 101/32, A23L 29/10

(54) **METHOD FOR SEPARATING THE SURFACTANTS PRESENT IN THE WASHING LIQUORS OF CORN, AND USES**
VERFAHREN ZUR TRENNUNG DER TENSIDE IN WASCHLAUGEN VON MAIS UND VERWENDUNGEN
PROCÉDÉ DE SÉPARATION DES SURFACTANTS PRÉSENTS DANS LES LIQUEURS DE LAVAGE DE MAÏS ET UTILISATIONS

(30) Priority: 18.06.2012 ES 201200649
(43) Date of publication of application: 22.04.2015
(73) Proprietor: Universidad de Vigo, 36310 Vigo (ES)
(72) Inventor: MOLDES MENDUIÑA, Ana Belén, 36310 Vigo (ES); CRUZ FREIRE, José Manuel, 36310 Vigo (ES); DEVESA REY, Rosa, 36310 Vigo (ES); VECINO BELLO, Xanel, 36310 Vigo (ES)
(86) International application number: PCT/ES2013/000150
(87) International publication number: WO 2014/044876

(56) References cited:
- US-A- 2 444 176
- US-A- 2 477 763
- US-A- 2 712 516
- X. VECINO ET AL: "Optimization of liquid-liquid extraction of biosurfactants from corn steep liquor", BIOPROCESS AND BIOSYSTEMS ENGINEERING, vol. 38, no. 9, 26 April 2015 (2015-04-26) , pages 1629-1637, XP055230937, DE ISSN: 1615-7591, DOI: 10.1007/s00449-015-1404-9
- X. VECINO ET AL: "Study of the Surfactant Properties of Aqueous Stream from the Corn Milling Industry", JOURNAL OF AGRICULTURAL AND FOOD CHEMISTRY, vol. 62, no. 24, 18 June 2014 (2014-06-18) , pages 5451-5457, XP055230939, US ISSN: 0021-8561, DOI: 10.1021/jf501386h
- Parveen Jamal ET AL: "Optimization of Process Conditions For Extraction of Biosurfactant", Journal of Applied Sciences Research, 1 January 2011 (2011-01-01), pages 2156-2161, XP055230978, Retrieved from the Internet: URL:http://www.aensiweb.com/old/jasr/jasr/ 2011/2156-2161.pdf [retrieved on 2015-11-24]
- Nikhil S Shaligram ET AL: "Surfactin--a review on biosynthesis, fermentation, purification and applications", Food Technology and Biotechnology, 1 April 2010 (2010-04-01), page 119, XP055230983, Retrieved from the Internet: URL:http://connection.ebscohost.com/c/arti cles/52724418/surfactin-review-biosynthesi s-fermentation-purification-applications [retrieved on 2015-11-24]
- SAHARAN BS. ET AL.: 'A REVIEW ON BIOSURFACTANTS: FERMENTATION, CURRENT DEVELOPMENTS AND PERSPECTIVES.' GENETIC ENGINEERING AND BIOTECHNOLOGY JOURNAL vol. 2011, no. GEBJ29, 2011, XP055175498 Retrieved from the Internet: <URL:htt :l/astonjournals.com/manuscripts/Accepted/G EBJ-29acc7-11-11.pdf> [retrieved on 2013-06-07]
- THROCKMORTON, P. ET AL.: 'Biodegradable surfactants derived from corn starch.' JOURNAL OF THE AMERICAN OIL CHEMIST SOCIETY vol. 51, no. 11, 1974, pages 486 - 494, XP001390300
- HEIMBUCH, A.H. ET AL.: 'SOME CHARACTERISTICS OF A GROWTH STIMULANT IN CORN STEEP LIQUOR FOR LACTOBACILLUS CASEI.' J. BACTERIOL. vol. 72, no. 4, 1956, pages 543 - 547, XP055175503

## Description

### Background of the invention

Surfactants are surface-active agents that can reduce the surface tension between two liquids. They contain both hydrophobic and hydrophilic groups, which enables them to occupy a position at the interface between liquids with different degrees of polarity (water/oil and oil/water) thus forming a film and reducing the surface tension of the mixture. A substance or product is considered to have the capacity to act as a surfactant when it can reduce the surface tension of water by 8 units (Busscher H., (1994). Appl. Microbiol. Biot. 41, 4-7). However, a product or compound is considered a good surfactant when it can reduce the surface tension of water by more than 20 units (Mulligan C., Gibbs B., (1993). Biosurfactants: production Properties and Applications. Marcel Dekker, New York, 329-371.). Surfactants have a large number of industrial applications and are used as detergents, emulsifiers, lubricating and foaming agents, as well as to solubilize and disperse liquid phases.

Conventional surfactants are synthetic. However, many surfactants are produced by living organisms, such as plants, microorganisms and even humans. These compounds, which are considered natural surfactants and are referred to as biosurfactants, may be more effective than synthetic surfactants. The advantages of biosurfactants obtained from natural sources include the following: a) Lower concentrations of these compounds are required to reduce the surface tension of a liquid or mixture of liquids. b) They work under a wider range of pH, temperature and ionic strength and can therefore be used in a wider range of conditions. c) They are biodegradable and therefore do not cause environmental problems. d) They display low toxicity and can therefore be safely used in the pharmaceutical, cosmetics and food industries (Desai, J.D., Banat, I.M., (1997). Microbiol. Mol. Biol. R. 61 (1), 47-64.).

Biosurfactants have numerous applications (Banat, I.M., (2000). Water Studies. 8, 177-185.): e.g., in **the food industry** biosurfactants are used to improve the rheological properties of products by favouring the formation of emulsions. Another area (also in the food sector) in which biosurfactants may play an important role is in the production of active packaging systems. These systems prevent pathogenic microorganisms adhering to their surface, thereby increasing the shelf life of foodstuffs. The antiadhesive property of biosurfactants has been demonstrated in various research studies (e.g. Gudiña, E. J., (2010). Lett. Appl. Microbiol. 50 (4), 419-424).

**Personal hygiene and cosmetic products.** Biosurfactants can be used in all types of personal hygiene and cosmetic products because of their good compatibility with skin. Production of biosurfactants by the action of enzymes (mainly lipases) on hydrophobic molecules has led to new applications for these compounds, mainly in the manufacture of personal hygiene products and cosmetics.

**Therapeutic applications.** One of the most important applications for biosurfactants is in the pharmaceutical industry (Rodrigues, L., (2004). Appl. Microbiol. Biot. 66 (3), 306-311). Surfactin, one of the best known biosufactants, has several medical-pharmaceutical applications of interest. These include the inhibition of clot formation, formation of ion channels in membranes, and as antibacterial, antifungal, antiviral and antitumor agents.

**Biosurfactants in agriculture.** Biosurfactants are mainly used in agriculture to produce herbicides and pesticides. The active components of such formulations are usually hydrophobic, and emulsifying agents are usually required to disperse the components in aqueous solutions.

**Bioremediation.** Biosurfactants can reduce the impact caused by the release of diverse compounds, such as hydrocarbons, to the environment (Moldes, A. B., (2011). J. Agr. Food Chem. 59 (17), 9443-9447). Many microorganisms are capable of degrading long-chain hydrocarbons, such as petroleum, although the hydrocarbons must be bioavailable for this to occur. Most hydrocarbons that contaminate the environment are highly insoluble in water. They often form hydrophobic bonds with certain types of surfaces, such as rock surfaces, and are therefore very difficult to remove because the microorganisms that could degrade them cannot gain access to them (i.e. the hydrocarbons are not bioavailable). Biosurfactants act by producing emulsions or by solubilizing the hydrocarbons or other water-insoluble compounds, thus increasing their bioavailability. Researchers in the field of bioremediation are seeking to discover microorganisms that produce biosurfactants that favour the bioavailability of hydrocarbons. Biosurfactants are also useful for bioremediation of environmental contamination with toxic heavy metals such as uranium, cadmium and lead.

Corn steep liquor is generated as a by-product in the corn wet-milling industry. It is produced when grains of maize are soaked in hot water to swell them and extract starch (US Patent, 4,980,282). This liquid contains low amounts of reducing sugars and also low quantities of free lysine, histidine, arginine, aspartic acid and tyrosine. It also contains large quantities of lactic acid (US Patent 4,980,282). The use of corn steep liquor as a nutritional supplement has been widely demonstrated in recent years. It can be used to replace expensive nutrients, such as yeast extract and peptone, in fermentation processes and in the production of alcohols, lipases, lactic acid and malic acid, amongst other products (Rivas B., (2004). Int. J. Food Microbiol. 97 (1), 93-98). This has lowered the cost of obtaining some biotechnological products.

### State of the art

The annual sales of surfactants are valued at around 23.9 million US dollars (Market Report, (2008). World Surfactant Market: markets, products, applications, innovations, chances & risks, competition, prospects to 2015, Germany: Active Market Intelligence.), with a global production close to 13 million tonnes (Rust D., (2008). Surfactants: a market opportunity study update, Midland, MI: OmniTech International Ltd; Levison M. I., (2009). Surfactant production: present realities and future perspectives. In: U. Zoller (ed) Handbook of detergents part F: production, Boca Raton, FL: 142, 1-38.). However, most of these surfactants are not biodegradable and may generate environmental problems when used for certain purposes, such as to treat contaminated soils or water. Production of biosurfactants that are biodegradable and do not degrade the environment is therefore of great interest. Although some microorganisms can produce biosurfactants, industrial production has not been fully developed owing to the high costs associated with the production and purification processes (WO2006136178 (A1), JP2003320367 (A)).

Until now, no studies have been carried out either to evaluate the capacity of corn steep liquor as source of tenso-active agents (biosurfactants) or to extract such agents from this waste liquid.

Here, we propose a method for extracting compounds from corn steep liquor for use as tenso-active agents, surfactants or detergents in different fields (in the food, cosmetics and pharmaceutical industries, for bioremediation of contaminated water and soils, and for agriculture applications).

### Description of the invention

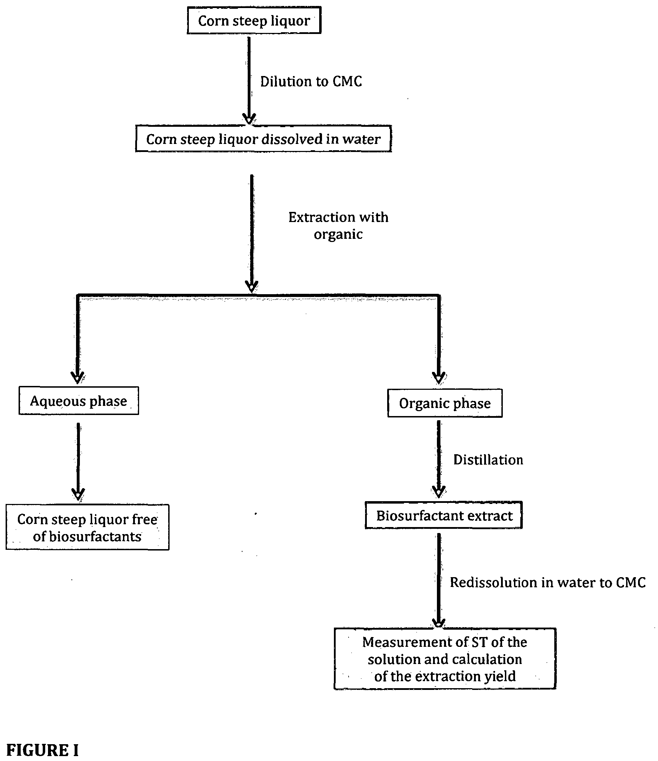

The protocol followed is illustrated in the diagram in **Figure 1****.**

### Extraction of biosurfactant extracts from corn steep liquor

Serial dilutions of corn steep liquor (SIGMA ALDRICH, 50% solids) were made to 20 g/L, to enable calculation of the critical micellar concentration (CMC) of the solution. The CMC of a detergent or biosurfactant is defined as the concentration at which micelles are formed and all additional surfactants added to the system go to micelles, thus the surface tension of water cannot be reduced any further.

After diluting the corn steep liquor, samples of the solutions were treated with different extractants (isoamyl alcohol; chloroform; ethyl acetate; tributyl phosphate (TBP); methyl butyl ether (MTBE); trichloroethylene (TCE); dichloromethane; xylene; hexane; and heptane). On completion of the extraction, the organic and aqueous phases were separated. The organic phase, which contains the extract of biosurfactant, was then distilled to remove the organic solvent used during the extraction. The biosurfactant extracts were then redissolved in water until reaching the initial volume of the corn steep liquor, to enable calculation of the extraction yield. Thus, the extraction yield was calculated on the basis of the increase in surface tension of this solution relative to the initial surface tension of the corn steep liquor (36±2 mN/m). The maximum value that the surface tension can reach after extraction and redissolution of the compounds in water is 72 mN/m - which would indicate that no biosurfactants had been extracted. The minimum value of surface tension that can be reached is 36±2 mN/m, which would indicate that 100% of the biosurfactants had been extracted.

### Evaluation of the capacity of the extracted compounds to act as tenso-active agents (surfactants)

The biosurfactant extracts obtained from the corn steep liquor were redissolved in water, and the surface tension (ST) was measured with a KRUSS K6 tensiometer, fitted with a platinum ring (1.9 cm Du Noüy). The surface tension of the prepared solution decreased by 36 units relative to that of water.

### Working example of use of the invention

### Extraction of the biosurfactant extracts with methyl butyl ether or chloroform

An aqueous solution of corn steep liquor was extracted with methyl butyl ether or chloroform (ratio of aqueous phase:organic phase, 5:10; shaking speed, 150 rpm; room temperature up to 4 hours). On completion of the extraction, the organic phase and the aqueous phase were separated. The organic phase was then distilled at a temperature higher than the boiling point of methyl butyl ether or chloroform to recover the organic dissolvent and to obtain an extract with surfactant properties. The extraction yield was 100% with both methyl butyl ether and with chloroform.

## Claims

1. Procedure for separation of the surfactants contained in corn steep liquor, which is **characterized by** the following steps; a) dilution of the corn steep liquor in water, b) extraction of the corn steep liquor with an organic solvent or a mixture of organic solvents, c) phase separation, and d) distillation of the volatile components of the organic phase.

2. Procedure for separation of the surfactants contained in corn steep liquor, according to claim 1, characterized for concentrations of the solution of corn steep liquor in water in step a) of between 5 g /L and 25 g/L.

3. Procedure for the separation of the surfactants contained in corn steep liquor, according to claim 1, characterized for different organic solvents used in extraction step b): isoamyl alcohol, chloroform, 1,2-dichloroethane, ethyl acetate, tetrahydrofurane, tributyl phosphate, methyl butyl ether, diethylether, trichloroethene, hexane, heptane, xylene, dichloromethane, or any mixture of these.

4. Use of the biosurfactant extract obtained by the procedure according to claims 1-3, as a tenso-active agent, detergent or surfactant.

5. Use of the biosurfactant extract obtained by the procedure according to claims 1-3, as a tenso-active agent, detergent or surfactant for the treatment of water or soils contaminated with hydrophobic substances.

6. Use of the biosurfactant extract obtained by the procedure according to claims 1-3, as a tenso-active agent, detergent or surfactant to improve the rheological properties of food products.

7. Use of the biosurfactant extract obtained by the procedure according to claims 1-3, as a tenso-active agent, detergent or surfactant in the formulation of pharmaceutical or cosmetic products.

8. Use of the biosurfactant extract obtained by the procedure according to claims 1-3, as a tenso-active agent, detergent or surfactant in the formulation of herbicides and pesticides.

## Patentansprüche

1. Verfahren zur Trennung der Tenside, die im Maisquellwasser enthalten sind, **gekennzeichnet durch** die folgenden Schritte; a) Verdünnung von Maisquellwasser in Wasser, b) Extraktion des Maisquellwassers mit einem organischen Lösungsmittel oder einer Mischung von organischen Lösungsmitteln, c) Phasentrennung, und d) Destillation der volatilen Komponente der organischen Phase.

2. Verfahren zur Trennung der Tenside, die im Maisquellwasser enthalten sind, gemäß Anspruch 1, **gekennzeichnet durch** Konzentrationen der Lösung von Maisquellwasser im Wasser gemäß Schritt a) von zwischen 5 g/l und 25 g/l.

3. Verfahren zur Trennung der Tenside, die in Maisquellwasser enthalten sind, gemäß Anspruch 1, **gekennzeichnet durch** verschiedene organische Lösungsmittel, die beim Extraktionsschritt b) verwendet werden: Isoamylalkohol, Chloroform, 1,2-Dichlorethan, Ethylacetat, Tetrahydrofuran, Tributylphosphat, Methylbutylether, Diethylether, Trichlorethen, Hexan, Heptan, Xylol, Dichlormethan, oder irgendeine Mischung davon.

4. Verwendung des Bio-Tensid-Extrasts, das bei dem Verfahren gemäß der Ansprüche 1-3 erhalten wurde, als oberflächenaktive Substanz, Reinigungsmittel oder Tensid.

5. Verwendung des Bio-Tensid-Extrakts, das bei dem Verfahren gemäß der Ansprüche 1-3 erhalten wurde, als oberflächenaktive Substanz, Reinigungsmittel oder Tensid für die Aufbereitung von Wasser oder Böden, die mit hydrophoben Substanzen verunreinigt wurden.

6. Verwendung des Bio-Tensid-Extrakts, das bei dem Verfahren gemäß der Ansprüche 1-3 enthalten wurde, als oberflächenaktive Substanz, Reinigungsmittel oder Tensid, um die rheologischen Eigenschaften von Nahrungsmitteln zu verbessern.

7. Verwendung des Bio-Tensid-Extrakts, das bei dem Verfahren gemäß der Ansprüche 1-3 enthalten wurde, als oberflächenaktive Substanz, Reinigungsmittel oder Tensid bei der Formulierung von pharmazeutischen oder kosmetischen Produkten.

8. Verwendung des Bio-Tensid-Extrakts, das bei dem Verfahren gemäß der Ansprüche 1-3 enthalten wurde, als oberflächenaktive Substanz, Reinigungsmittel oder Tensid bei der Formulierung von Unkraut- und Schädlingsbekämpfungsmitteln.

## Revendications

1. Procédé de séparation des surfactants contenus dans la liqueur de lavage de maïs, **caractérisé par** les étapes suivantes ; a) dilution de la liqueur de macération de maïs dans l'eau, b) extraction de la liqueur de macération de maïs avec un solvant organique ou un mélange de solvants organiques, c) séparation des phases et d) distillation des composants volatils de la phase organique.

2. Procédé de séparation des surfactants contenus dans la liqueur de lavage de maïs, conformément à la revendication 1, **caractérisé par** des concentrations de la solution de liqueur de macération de maïs dans l'eau à l'étape a) comprise entre 5 g/L et 25 g/L

3. Procédé de séparation des surfactants contenus dans la liqueur de lavage de maïs, conformément à la revendication 1, **caractérisé par** différents solvants organiques utilisés dans l'étape d'extraction b) : alcool isoamylique, chloroforme, 1,2-dichloroéthane, acétate d'éthyle, tétrahydrofurane, phosphate de tributyle, méthyle L'éther butylique, l'éther diéthylique, le trichloroéthène, l'hexane, l'heptane, le xylène, le dichlorométhane ou tout mélange de ceux-ci.

4. Utilisation de l'extrait de biosurfactant obtenu par le procédé selon les revendications 1 à 3, comme agent tensioactif, détergent ou surfactant.

5. Utilisation de l'extrait de biosurfactant obtenu par le procédé selon les revendications 1 à 3, comme agent tensioactif, détergent ou surfactant pour le traitement de l'eau ou des sols contaminés par des substances hydrophobes.

6. Utilisation de l'extrait de biosurfactant obtenu par le procédé selon les revendications 1 à 3, comme agent tensioactif, détergent ou surfactant pour améliorer les propriétés rhéologiques des produits alimentaires.

7. Utilisation de l'extrait de biosurfactant obtenu par le procédé selon les revendications 1 à 3, comme agent tensioactif, détergent ou surfactant dans la formulation de produits pharmaceutiques ou cosmétiques.

8. Utilisation de l'extrait de biosurfactant obtenu par le procédé selon les revendications 1 à 3, comme agent tensioactif, détergent ou surfactant dans la formulation des herbicides et pesticides.
